# EUROPEAN PATENT SPECIFICATION

(11) **EP 0 542 999 B1**
(45) Date of publication and mention of the grant of the patent: **03.09.1997**
(21) Application number: 92913900.4
(22) Date of filing: 05.06.1992
(51) Int. Cl.: A61M 25/00

(54) **UNCOLLAPSIBLE CATHETER**
NICHT-FALTBARER KATHETER
CATHETER NE POUVANT PAS SE PLIER

(30) Priority: 07.06.1991 US 712033
(43) Date of publication of application: 26.05.1993
(73) Proprietor: TAUT, INC., Geneva, IL 60134 (US)
(72) Inventor: McFARLANE, Richard, Harper, Geneva, IL 60134 (US)
(74) Representative: Spall, Christopher John
(86) International application number: PCT/US92/04669
(87) International publication number: WO 92/21398

(56) References cited:
- DE-U- 8 804 345
- SU-A- 940 777
- US-A- 3 757 768
- US-A- 3 780 740
- US-A- 3 894 541
- US-A- 4 306 566
- US-A- 4 547 192
- US-A- 4 719 924
- US-A- 4 737 153
- US-A- 5 017 193
- US-A- 5 069 217

## Description

### BACKGROUND OF THE INVENTION

### Field of the Invention

This invention relates to catheters and use methods. In particular, it relates to catheters used in association with relatively tight clamping means for preventing escape of dyes and other substances around outside surfaces of the catheters when in use.

### Description of the Related Art

In current endoscopic procedures and related use of dyes in association with catheters for examining and treating such hollow anatomical organs as the gallbladder through the cystic duct, there exists a very serious problem of inadvertently including the duct and a catheter inserted into it with clamping pressure of clipping tools for positioning and fastening ligation clips to seal fluid flow around the catheter. One U.S. surgical product used, for instance, is known as a Sutiate Titanium Clip. It is held and position around the duct by a very thin-bodied special clipping instrument. Its use resembles that of a staple in a stapler. The jaws of the clipping instrument are very small in proportion to length and size of its handle. This causes high leverage and mechanical advantage that make it difficult to determine how tightly the clip is being fastened around a duct in which a catheter is inserted. Consequently, in a lot of cases, inclusion results from squeezing the duct and the catheter together.

In the medical field where lives are at risk and losses from errors are immense, this is a very serious problem. It has gone unsolved for decades. Now with the use of endoscopic equipment and associated treatment procedures, the problem has become increasingly acute, owing to related incision confinement. A wide variety of similar problems have led to this invention.

Su 940-777 discloses a therapeutic blockage catheter made of an elastic material with a closed working end, of similar diameter to that of the remainder of the catheter. X-ray contrastive ring markers are positioned inside the working end, and provide sufficient tension to reinforce the catheter, preventing it from flattening out on insertion into a pain-killing blockage site. The width of the markers and the distance between them provide sufficient rigidity while preserving the elasticity of the working end. In use, the catheter is introduced into the blockage site, and anaesthetic injected under pressure into the working end. Side apertures are provided in the working end, and the anaesthetic flows through these into the blockage site. It can be seen that, depending on the site, anaesthetic may flow away from the working end in either direction, i.e. it may flow back along the outside of the length of the catheter inserted in the site.

### Summary of the Invention

According to the present invention, there is provided an uncollapsible catheter having a distal section comprising an elongate, resilient tube section having a generally uniform interior and exterior diameter, and a head portion, and an internally positioned collapse inhibitor, said collapse inhibitor being a select length so as to define an uncollapsible section extending generally from said head and in said tube section, wherein said head portion is of a generally larger exterior diameter than said tube section, and is integrally formed therewith so as to form a rounded shoulder therebetween.

The collapse inhibitor can be metallic, preferably a stainless steel or titanium alloy. It can be short enough to allow bending of the catheter at a select distance from the tip or head. There can be a plurality of short, flat or round collapse inhibitors with bending flexibility of the catheter between them. The collapse inhibitor can also be a continuous linear coil. A use includes tightly positioning a clip around an anatomical duct in which an uncollapsible catheter is inserted without peril of squeezing the catheter and the duct together.

### Brief Description of the Drawings

This invention is described in appended claims in relation to description of a preferred embodiment with reference to the following drawings wherein:
Figure 1 is a cutaway side view of the invention being used in a cystic duct to prevent catheter and duct inclusion by clamping pressure of a duct clip.
Figure 2 is a front elevation view of a convention Sutiate Titanium Clip used with special insertion tools to seal ducts around inserted catheters.
Figure 3 is a cutaway side view of an embodiment of this invention with a solid tube positioned in a section of catheter adjacent to a tip portion of the catheter.
Figure 4 is a cutaway side view of the invention with a plurality of solid tubes, some with optional rounded edges for facilitating movement against each other.
Figure 5 is a cutaway side view of the invention using a series of square-walled rings positioned in the catheter to prevent its collapse while allowing it to bend.
Figure 6 is a cutaway side view of the invention using a series of round-walled rings positioned in the catheter to prevent its collapse while allowing it to bend.
Figure 7 is a cutaway side view of the invention using a square-walled metal coil positioned in the catheter to prevent its collapse when used in conjunction with a sutiate clip.
Figure 8 is a cutaway side view of the invention using a round-walled metal coil positioned in the catheter to prevents its collapse and having a smooth tubular base in which the coil is embedded to prevent entrapment of material in the catheter.

### Description of the Preferred Embodiment

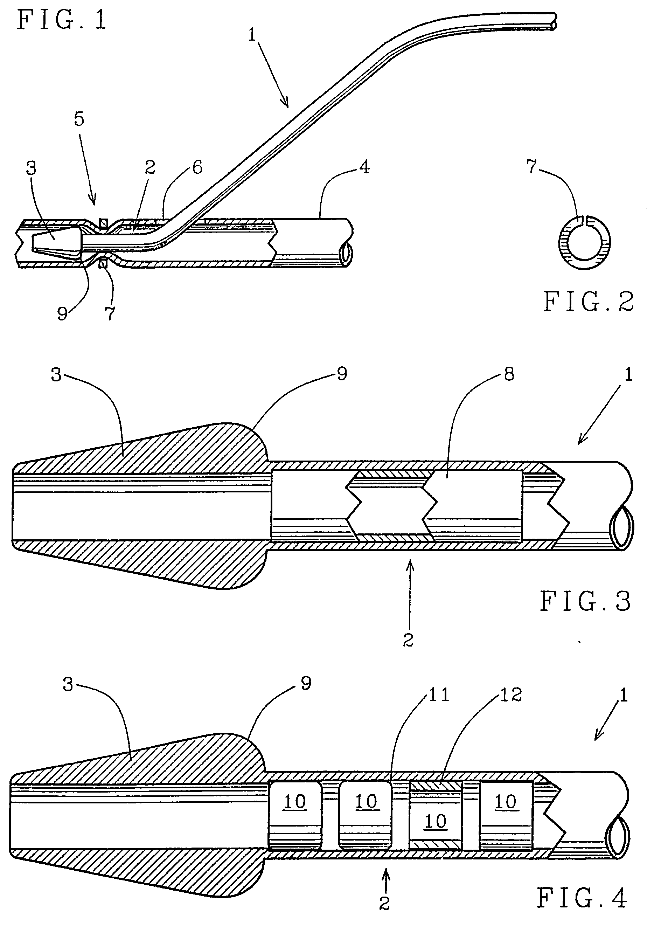

Referring to Figure 1 of the drawings, a catheter 1 with an uncollapsible section 2 in close proximity to a catheter head 3 is positionable in an anatomical duct 4, such as a cystic duct, at a sealing section 5 of the duct 4. For inserting a catheter in an anatomical duct 4 such as cystic duct without a natural outside entrance, an incision 6 is made and the catheter 1 is bent as necessary after insertion to allow the catheter 1 to be a different attitudinal angle at the head 3 than the remaining portion of the catheter 1.

Current procedures in the use of catheters include insertion of dyes in anatomical ducts 4 through the catheters 1. Distribution of the dyes in body organs is then observed with endoscopic instruments which are often inserted through the catheter. This endoscopic procedure is in lieu of observation with more open surgery and with other equipment such as X-rays. Objectives usually are to determine the existence, nature and extent of cystic calculus, adenomas, Morgagnian cataracts, degeneration, malignancy, adenoma destruens and other dysfunctions.

In order to achieve an effective level of disbursement of dyes and other agents in body organs, it is helpful to employ a select level of pressure against them through the catheter 1. To prevent escape and passage of fluids past the catheter 1 and the catheter head 3 as a result of the pressure, a clamping or sealing device in the form of a clip 7, shown separately from a face view in Figure 2, is inserted around the duct 4 and bent or otherwise fastened. Clamping pressure of a tool used to insert the clip 7 often closes the duct 4 and the catheter 1 inadvertently. Collapse or closure of the catheter 1 can impair its use and effectiveness for fully achieving objectives of an operational procedure.

In Figure 3, a relatively solid collapse inhibitor 8 is shown inside the catheter 1 which may include an internal, smooth, non-porous base, wherein the collapse inhibitor 8 is positioned in select proximity to the catheter head 3. The collapse inhibitor 8 can be in the form of a hollow metallic cylinder approximately the length of an average conventional catheter head and may include a non-metallic coating thereon to minimize possible corrosion of the metal. The term "uncollapsible" used in relation to this invention is relative to resistance of pressure application to a fastening clip 7.

Conventional catheter heads often have abrupt flash shoulders at their major diameter ends. This aids in providing a seal without applying enough clamping pressure on the clip 7 to close the conventional catheter. But it is difficult and highly user-dependent to be able to apply only enough clamping pressure on the clip 7 for preventing leakage around the conventional catheter without closing it and rendering it useless for achieving desired objectives. Often an entire operation fails when a conventional catheter collapses from this clamping pressure. A patient's life may be imperiled and observations may be misleading or incomplete. Further, when the conventional catheter with abrupt seal-inducing shoulders is removed, it can injure linings of anatomical ducts and organs. With this invention, however, it is not necessary to achieve sealing with a sharp-edged catheter head. Instead, a selectively rounded catheter shoulder 9 is used because the uncollapsible section 2 of the catheter 1 will not collapse when a clip 7 is tightened on the duct 4 sufficiently tight to prevent leakage from an upstream portion of the duct 4.

Reference is made now to Figure 4. In order to provide an uncollapsible section 2 of a catheter 1 close to and also further removed from a catheter head 3, a plurality of short collapse inhibitors 10 can be employed. This increases flexibility in that a clip 7 can be positioned without probability of failure of the catheter function. Rounded corners 11 can be provided on the short collapse inhibitors 10 in order for them to allow bending of the uncollapsible section 2 when they may be positioned relatively close together. These short collapse inhibitors 10 have walls with rectangular cross sections 12.

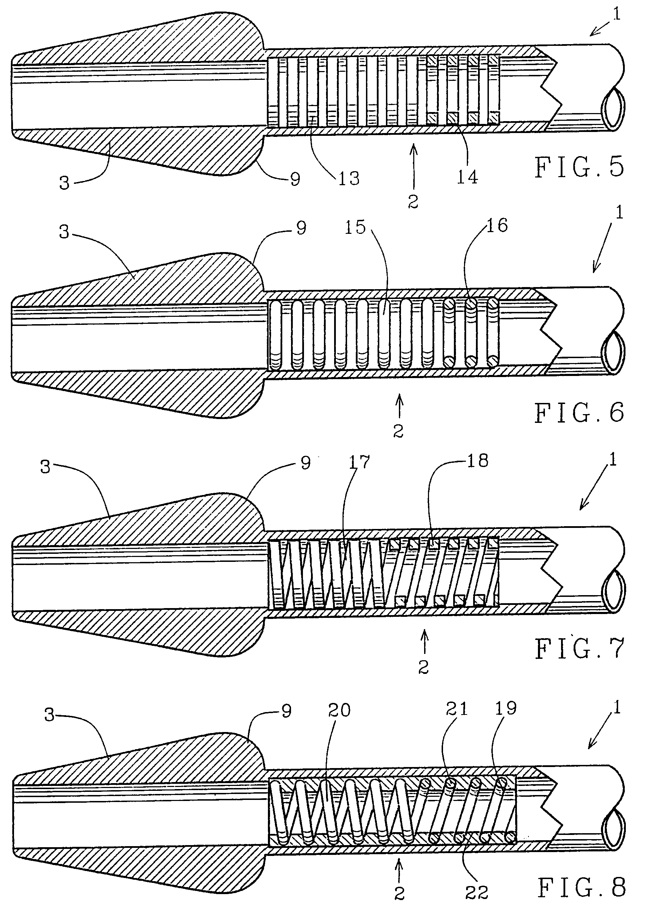

In Figure 5, a plurality of Square-walled rings 13 are employed as a collapse inhibitor for construction of the uncollapsible section 2. Each square-walled ring 13 has an effectively square cross section 14. These increase further yet the flexibility of where the uncollapsible section 2 can be bent for fitting a catheter into a duct 4 from an attitudinal angle different from linear axis of the duct 4.

In Figure 6, a plurality of round-walled rings 15 with substantially round cross sections 16 are employed to add flexibility of the uncollapsible section 2. Roundness of edges allows the rings 16 to be positioned closer yet together while allowing ease of bending without collapse from sealing pressure on the uncollapsible section 2.

Figure 7 shows use of a square-walled coil 17 with square cross sections 18 embedded in the inside periphery 19 of the uncollapsible section 2.

Figure 8 is a round-walled coil 20 with round cross sections 21 employed to provide uncollapsibility. The round crops sections 21 are embedded in a smooth base 22 to avoid areas for accumulation of contaminants around surfaces of the round-walled coil 20.

The smooth base 22 can be employed with any of the collapse inhibitors 8, 10, 13, 15, 17 or 20. To avoid conditions for contamination further, material for construction of the collapse inhibitors 8, 10, 13, 15, 17 or 20 can be stainless steel with either of a wide variety of alloys of nickel and chromium. Titanium and its alloys also are particularly noncorrosive. Alloys of palladium and titanium are relatively inexpensive and easily formable and machinable in comparison to other titanium metals. In addition to avoiding contamination conditions by being stainless and noncorrosive, titanium alloys are only about a third as heavy per strength of collapse resistance for this invention.

In selecting between square-walled coils 17 and round-walled coils 20, tradeoff factors are greater strength with less diameter per strength of the square cross sections 18 and greater catheter-bending characteristics with the round cross sections 21. These same tradeoff factors apply for square-walled rings 13 and round-walled rings 15.

An entire length of catheter 1 can be constructed with either collapse inhibitors 13, 15, 17 or 20. Most convenient for construction would be square-walled coils 17 or round-walled coils 18.

A new and useful uncollapsible catheter has been described. All such modifications, adaptations, applications and forms of the catheter as described by the following claims are included in this invention.

## Claims

1. An uncollapsible catheter (1) having a distal section comprising:
an elongate, resilient tube section having a generally uniform interior and exterior diameter, and a head portion (3), and
an internally positioned collapse inhibitor (8,10,13,15,17,20), said collapse inhibitor being a select length so as to define an uncollapsible section (2) extending generally from said head (3) and in said tube section,
characterised in that:
said head portion (3) is a of a generally larger exterior diameter than said tube section, and is integrally formed therewith so as to form a rounded shoulder (9) therebetween.

2. An uncollapsible catheter (1) according to claim 1 wherein the uncollapsible section (2) is provided with a metallic-tube collapse inhibitor (8) fixably positionable in the uncollapsible section (2) of the catheter (1).

3. An uncollapsible catheter (1) according to claim 2 and further comprising non-metallic material coating on the interior and end surfaces of the metallic-tube collapse inhibitor (8).

4. An uncollapsible catheter (1) according to claim 1 wherein the uncollapsible section (2) is positioned at a select distance from the catheter head (3) to allow bending of the catheter (1) at both ends of the uncollapsible section (2).

5. An uncollapsible catheter (1) according to claim 1 and further comprising a plurality of short collapse inhibitors (10) of select lengths fixably positionable at select distances from each other within the catheter (1).

6. An uncollapsible catheter (1) according to claim 5 wherein the plurality of short collapse inhibitors (10) are separate rings (13,15) with rigid bores of select lengths.

7. An uncollapsible catheter (1) according to claim 6 wherein the separate rings (13,15) are of substantially equivalent dimensions.

8. An uncollapsible catheter (1) according to claim 7 wherein the separate rings (13,15) are positionable with a distance between them which allows select bending of the catheter (1).

9. An uncollapsible catheter (1) according to claim 6 or claim 7 wherein said separate rings (15) include rounded exterior surfaces.

10. An uncollapsible catheter (1) according to claim 1 including a truncated-cone head (3) having selectively rounded edges (9) on a major-diameter thereof.

11. An uncollapsible catheter (1) according to claim 1 wherein said collapse inhibitor includes a linear resilient coil (17,20) fixably positionable within the catheter (1) and linear to its axial walls.

12. An uncollapsible catheter (1) according to claim 11 wherein the resilient linear coil (17,20) is a metallic coil spring.

13. An uncollapsible catheter (1) according to claim 12 wherein a cross-section (18) of a coiled wire forming the metallic coil spring (17) is rectangular.

14. An uncollapsible catheter (1) according to claim 12 wherein a cross-section (21) of a coiled wire forming the coil spring (20) is round.

15. An uncollapsible catheter (1) according to claim 11 wherein the resilient linear coil (17,20) is positionable in select proximity to said head (3) of the catheter (1) and extended a select distance therefrom within the catheter (1).

16. An uncollapsible catheter (1) according to claim 1 and further comprising:
a plurality of collapse inhibiting members (13,15,17,20) of select dimension linear to the catheter (1) extended a select distance throughout the catheter (1) from the catheter head (3); and
a smooth, non-porous base (22) in which the plurality of collapse inhibitors (13,15,17,20) are positioned to prevent accumulation of contamination around the collapse inhibitors (13,15,17,20).

## Patentansprüche

1. Unnachgiebiger Katheter (1) mit einem distalen Abschnitt, umfassend:
einen länglichen, federelastischen Schlauchabschnitt mit einem etwa gleichmäßigen Innen- und Außendurchmesser und einem Kopfabschnitt (3), und
eine intern angeordnete Einschnürsperre (8, 10, 13, 15, 17, 20), die eine solche ausgewählte Länge besitzt, daß ein unnachgiebiger Abschnitt (2) gebildet wird, welcher sich etwa von dem Kopf (3) ausgehend und in dem Schlauchabschnitt erstreckt,
**dadurch gekennzeichnet**, daß
der Kopfabschnitt (3) einen allgemein größeren Außendurchmesser als der Schlauchabschnitt besitzt und mit diesem einstückig ausgebildet ist, um dazwischen eine abgerundete Schulter (9) zu bilden.

2. Katheter (1) nach Anspruch 1, bei dem der urnachgiebige Abschnitt (2) mit einer als Metallröhrchen ausgebildeten Einschnürsperre (8) ausgestattet ist, die fixierbar in dem unnachgiebigen Abschnitt (2) des Katheters (1) positionierbar ist.

3. Katheter (1) nach Anspruch 2, umfassend einen aus nicht-metallischem Material bestehenden Überzug auf der Innenfläche und den Stirnflächen der als metallisches Röhrchen ausgebildeten Einschnürsperre (8).

4. Katheter (1) nach Anspruch 1, bei dem der unnachgiebige Abschnitt (2) einen gewählten Abstand von dem Katheterkopf (3) aufweist, um den Katheter (1) an beiden Enden des unnachgiebigen Abschnitts (2) durchbiegen zu können.

5. Katheter (1) nach Anspruch 1, umfassend mehrere kurze Einschnürsperren (10) mit jeweils vorbestimmter Länge, die in wählbaren Abständen voneinander innerhalb des Katheters (1) fest positionierbar sind.

6. Katheter (1) nach Anspruch 5, bei dem die mehreren kurzen Einschnürsperren (10) separate Ringe (13, 15) sind, die starre Bohrungen vorbestimmter Länge besitzen.

7. Katheter (1) nach Anspruch 6, bei dem die separaten Ringe (13, 15) im wesentlichen äquivalente Abmessungen besitzen.

8. Katheter (1) nach Anspruch 7, bei dem die separaten Ringe (13, 15) mit einem Zwischenabstand angeordnet sind, der ein selektives Biegen des Katheters (1) gestattet.

9. Katheter (1) nach Anspruch 6 oder 7, bei dem die separaten Ringe (15) abgerundete Außerflächen aufweisen.

10. Katheter (1) nach Anspruch 1, umfassend einen kegelstumpfförmigen Kopf (3) mit selektiv abgerundeten Rändern (9) an seinem Hauptdurchmesser.

11. Katheter (1) nach Anspruch 1, bei dem die Einschnürsperre eine lineare federelastische Spule (17, 20) aufweist, die innerhalb des Katheters (1) und linear zu dessen axialen Wänden fest positionierbar ist.

12. Katheter (1) nach Anspruch 11, bei dem die federelastische lineare Spule (17, 20) eine Metall-Schraubenfeder ist.

13. Katheter (1) nach Anspruch 12, bei dem ein Querschnitt (18) eines die metallische Schraubenfeder (17) gebildeten gewendelten Drahts rechteckig ist.

14. Katheter (1) nach Anspruch 12, bei dem ein Querschnitt (21) eines die Schraubenfeder (20) bildenden gewendelten Drahts rund ist.

15. Katheter (1) nach Anspruch 11, bei dem die federelastische lineare Spule (17, 20) in vorbestimmter Nähe zu dem Kopf (3) des Katheters (1) positionierbar ist und sich davon innerhalb des Katheters (1) um eine vorbestimmte Strecke ausdehnt.

16. Katheter (1) nach Anspruch 1, umfassend:
mehrere Einschnürsperrglieder (13, 15, 17, 20) vorbestimmter Abmessung entlang des Katheters (1), die sich über eine vorbestimmte Strecke von dem Katheterkopf (3) aus durch das Katheter (1) erstrecken; und
einen glatten, nicht-porösen Träger (22), in dem die Einschnürsperren (13, 15, 17, 20) angeordnet sind, um eine Ansammlung von Verunreinigungen um die Einschnürsperren (13, 15, 17, 20) herum zu unterbinden.

## Revendications

1. Cathéter ne pouvant pas être aplati (1) ayant une section distale comprenant :
- une section de tube élastique allongé ayant des diamètres, intérieur et extérieur, généralement uniformes et une portion de tête (3) ; et
- un inhibiteur d'aplatissement disposé à l'intérieur (8, 10, 13, 15, 17, 20), ledit inhibiteur d'aplatissement ayant une longueur sélectionnée de façon à définir une section ne pouvant pas être aplatie (2) s'étendant généralement depuis ladite tête (3) et dans ladite section de tube,
caractérisé en ce que :
ladite portion de tête (3) a un diamètre extérieur généralement plus grand que ladite section de tube, et est formé d'un seul tenant avec elle de façon à former entre elles un épaulement arrondi (9).

2. Cathéter ne pouvant pas être aplati (1) selon la revendication 1, dans lequel la section ne pouvant pas être aplatie (2) est équipée d'un inhibiteur d'aplatissement sous forme d'un tube métallique (8) pouvant être positionné de façon fixe dans la section ne pouvant pas être aplatie (2) du cathéter (1).

3. Cathéter ne pouvant pas être aplati (1) selon la revendication 2, comprenant en outre un revêtement d'un matériau non métallique sur les surfaces intérieure et terminale de l'inhibiteur d'aplatissement sous forme d'un tube métallique (8).

4. Cathéter ne pouvant pas être aplati (1) selon la revendication 1, dans lequel la section ne pouvant pas être aplatie (2) est positionnée à une distance choisie de la tête de cathéter (3) pour permettre de courber le cathéter (1) aux deux extrémités de la section ne pouvant pas être aplatie (2).

5. Cathéter ne pouvant pas être aplati (1) selon la revendication 1, comprenant en outre une multiplicité de courts inhibiteurs d'aplatissement (10) de longueur sélectionnée, pouvant être positionnés de façon fixe à des distances sélectionnées l'un de l'autre à l'intérieur du cathéter (1).

6. Cathéter ne pouvant pas être aplati (1) selon la revendication 5, dans lequel la multiplicité de courts inhibiteurs d'aplatissement (10) est constituée par des anneaux séparés (13, 15) avec des alésages rigides de longueurs sélectionnées.

7. Cathéter ne pouvant pas être aplati (1) selon la revendication 6, dans lequel les anneaux séparés (13, 15) ont des dimensions pratiquement équivalentes.

8. Cathéter ne pouvant pas être aplati (1) selon la revendication 7, dans lequel on peut positionner les anneaux séparés (13, 15) avec une distance entre eux qui permet une courbure sélectionnée du cathéter (1).

9. Cathéter ne pouvant pas être aplati (1) selon la revendication 6 ou la revendication 7, dans lequel lesdits anneaux séparés (15) ont des surfaces extérieures arrondies.

10. Cathéter ne pouvant pas être aplati (1) selon la revendication 1, comprenant une tête tronconique (3) ayant des bords sélectivement arrondis (9) sur son plus grand diamètre.

11. Cathéter ne pouvant pas être aplati (1) selon la revendication 1, dans lequel ledit inhibiteur d'aplatissement comprend un enroulement élastique linéaire (17, 20) pouvant être positionné de façon fixe à l'intérieur du cathéter (1) et aligné avec ses parois axiales.

12. Cathéter ne pouvant pas être aplati (1) selon la revendication 11, dans lequel l'enroulement linéaire élastique (17, 20) est un ressort hélicoïdal métallique.

13. Cathéter ne pouvant pas être aplati (1) selon la revendication 12, dans lequel la section transversale (18) du fil métallique enroulé formant le ressort hélicoïdal métallique (17) est rectangulaire.

14. Cathéter ne pouvant pas être aplati (1) selon la revendication 12, dans lequel la section transversale (21) du fil métallique enroulé formant le ressort hélicoïdal (20) est circulaire.

15. Cathéter ne pouvant pas être aplati (1) selon la revendication 11, dans lequel l'enroulement linéaire élastique (17, 20) peut être positionné à proximité sélectionnée de la tête (3) du cathéter (1) et s'étend sur une distance sélectionnée de la tête à l'intérieur du cathéter (1).

16. Cathéter ne pouvant pas être aplati (1) selon la revendication 1, comprenant en outre :
- une multiplicité d'éléments inhibiteurs d'aplatissement (13, 15, 17, 20) d'une dimension sélectionnée le long du cathéter (1), s'étendant sur une distance sélectionnée à travers le cathéter (1) depuis la tête de cathéter (3) ; et
- une base lisse non poreuse (22) dans laquelle la multiplicité d'inhibiteurs d'aplatissement (13, 15, 17, 20) sont positionnés pour empêcher une accumulation de contamination autour des inhibiteurs d'aplatissement (13, 15, 17, 20).
